(19) Europäisches Patentamt / European Patent Office / Office européen des brevets

(11) **EP 3 609 574 B1**

(12) **EUROPEAN PATENT SPECIFICATION**

(45) Date of publication and mention
of the grant of the patent:
**07.06.2023 Bulletin 2023/23**

(21) Application number: **18722327.6**

(22) Date of filing: **16.04.2018**

(51) International Patent Classification (IPC):
**A61N 5/06** *(2006.01)*    **A61N 5/067** *(2006.01)*
**A61N 1/40** *(2006.01)*    **A61N 2/00** *(2006.01)*
**A61N 7/02** *(2006.01)*    A61N 1/36 *(2006.01)*
**A61N 7/00** *(2006.01)*

(52) Cooperative Patent Classification (CPC):
**A61N 7/02; A61N 5/0622; A61N 5/067;**
A61N 1/36017; A61N 1/3606; A61N 1/40;
A61N 2005/0659; A61N 2007/0026

(86) International application number:
**PCT/US2018/027743**

(87) International publication number:
**WO 2018/191744 (18.10.2018 Gazette 2018/42)**

(54) **TARGETING SMALL-DIAMETER AXONS FOR NEUROMODULATION**

ABZIELUNG AUF AXONE MIT KLEINEM DURCHMESSER ZUR NEUROMODULATION

CIBLAGE D'AXONES DE FAIBLE DIAMÈTRE POUR NEUROMODULATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.04.2017 US 201762485660 P
03.04.2018 US 201862651975 P**

(43) Date of publication of application:
**19.02.2020 Bulletin 2020/08**

(73) Proprietors:
• **Case Western Reserve University
Cleveland, OH 44106 (US)**
• **Vanderbilt University
Nashville, TN 37240 (US)**
• **University of Pittsburgh - Of the Commonwealth
System of Higher Education
Pittsburgh, PA 15260 (US)**

(72) Inventors:
• **LOTHET, Emilie H.
Cleveland, OH 44106 (US)**
• **SHAW, Kendrick M.
Cleveland, OH 44106 (US)**
• **JANSEN, E. Duco
Cleveland, OH 44106 (US)**
• **CHIEL, Hillel J.
Cleveland, OH 44106 (US)**
• **JENKINS, Michael W.
Cleveland, OH 44106 (US)**
• **HORN, Charles
Pittsburg, PA 15260 (US)**

(74) Representative: **Marks & Clerk LLP
15 Fetter Lane
London EC4A 1BW (GB)**

(56) References cited:
**US-A1- 2004 015 204**    **US-A1- 2009 187 223**
**US-A1- 2014 074 176**    **US-A1- 2014 228 900**

• **LOTHET EMILIE H ET AL: "Selective control of
small versus large diameter axons using infrared
laser light(Conference Presentation)",
PROGRESS IN BIOMEDICAL OPTICS AND
IMAGING, SPIE - INTERNATIONAL SOCIETY FOR
OPTICAL ENGINEERING, BELLINGHAM, WA, US,
vol. 9690, 8 March 2016 (2016-03-08), pages
96901N-96901N, XP060066471, ISSN: 1605-7422,
DOI: 10.1117/12.2212945 ISBN:
978-1-5106-0027-0**

- ZHUO JUNQI ET AL: "Analysis of components of compound action potentials in response to infrared laser light (Conference Presentation)", PROGRESS IN BIOMEDICAL OPTICS AND IMAGING, SPIE - INTERNATIONAL SOCIETY FOR OPTICAL ENGINEERING, BELLINGHAM, WA, US, vol. 10052, 8 February 2017 (2017-02-08), pages 100520N-100520N, XP060084390, ISSN: 1605-7422, DOI: 10.1117/12.2253275 ISBN: 978-1-5106-0027-0
- ALEKSANDRA VUCKOVIC ET AL: "A comparative study of three techniques for diameter selective fiber activation in the vagal nerve: anodal block, depolarizing prepulses and slowly rising pulses; Anodal block, depolarizing prepulses and slowly rising pulses", JOURNAL OF NEURAL ENGINEERING, INSTITUTE OF PHYSICS PUBLISHING, BRISTOL, GB, vol. 5, no. 3, September 2008 (2008-09), pages 275-286, XP020147189, ISSN: 1741-2552, DOI: 10.1088/1741-2560/5/3/002
- SONG LUAN ET AL: "Neuromodulation: present and emerging methods", FRONTIERS IN NEUROENGINEERING, vol. 7, 15 July 2014 (2014-07-15), XP055489127, DOI: 10.3389/fneng.2014.00027
- EMILIE H. LOTHET ET AL: "Selective inhibition of small-diameter axons using infrared light", SCIENTIFIC REPORTS, vol. 7, no. 1, 12 June 2017 (2017-06-12), XP055489046, DOI: 10.1038/s41598-017-03374-9

**Description**

**Technical Field**

The present disclosure relates generally to neuromodulation and, more specifically, to systems that target small-diameter axons for neuromodulation

**Background**

Small-diameter axons play critical roles in sensory, autonomic, and motor systems. For example, small-diameter unmyelinated C-fibers carry nociceptive signals, while small-diameter unmyelinated motor axons are often involved in control of peripheral glands and other autonomic structures. Controlling the activity of these small-diameter axons can be used to treat medical conditions linked to the small-diameter axons. A theory of selective control of small versus large diameter axons using infrared laser light based on a mathematical analysis of the cable equation is described in LOTHET EMILIE H ET AL: "Selective control of small versus large diameter axons using infrared laser light (Conference Presentation)", PROGRESS IN BIOMEDICAL OPTICS AND IMAGING, SPIE - INTERNATIONAL SOCIETY FOR OPTICAL ENGINEERING, BELLINGHAM, WA, US, vol. 9690, 8 March 2016, page 96901 N, XP060066471, ISSN: 1605-7422, DOI: 10.1117/12.2212945 ISBN: 978-1-5106-0027-0. A numerical simulation for determining shifts in compound action potentials in response to infrared laser light is presented in ZHUO JLTNQI ET AL: "Analysis of components of compound action potentials in response to infrared laser light (Conference Presentation)", PROGRESS IN BIOMEDICAL OPTICS AND IMAGING, SPIE - INTERNATIONAL SOCIETY FOR OPTICAL ENGINEERING, BELLINGHAM, WA, US, vol. 10052, 8 February 2017, page 100520N, XP060084390, ISSN: 1605-7422, DOI: 10.1117/12.2253275, ISBN: 978-1-5106-0027-0. A system for suppression of neural activities of a target of interest that includes a laser source that may be used to target smaller diameter axons is described in US 2014/074176 A1.

US 2014/28900 A1 shows a system for selectively recruiting a nerve fiber type within nerve. US 2009/187223 A1 shows a system for selective nerve stimulation.

**Summary**

The present disclosure relates generally to neuromodulation and, more specifically, to systems that target small-diameter axons for neuromodulation Conduction of the small-diameter axons can be modulated to a greater degree to that of large-diameter axons in the target area. According to the present invention, there is provided a system as defined by claim 1. Preferred embodiments are defined by the dependent claims.

Aspects, embodiments, examples or methods described hereinafter which do not fall within the scope of the appended claims do not form part of the present invention and are presented for illustrative purposes.

We describe herein a method for targeting small-diameter axons for neuromodulation. This method is not part of the present invention and this method includes configuring a nerve modulating therapy. The nerve modulating therapy can be applied to a target area of a subject to modulate conduction in one or more small diameter axons within the target area to a greater degree than large diameter axons within the target area.

We also describe herein a system that targets small-diameter axons for neuromodulation. The system includes a source to provide a nerve modulating therapy. The system also includes a probe to apply the nerve modulating therapy to a target area of a subject to modulate conduction in one or more small diameter axons within the target area to a greater degree than large diameter axons within the target area.

**Brief Description of the Drawings**

The foregoing and other features of the present disclosure will become apparent to those skilled in the art to which the present disclosure relates upon reading the following description with reference to the accompanying drawings, in which:

FIG. 1 is a block diagram illustration showing an example of a system that configures and applies a nerve modulating therapy to target small-diameter axons for neuromodulation;

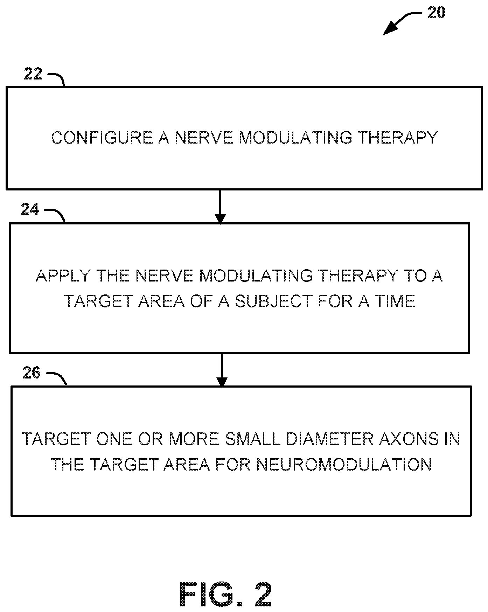

FIG. 2 is a process flow diagram of an example method for targeting small-diameter axons for neuromodulation;
FIG. 3 is an illustration of an experimental setup for selective optical inhibition;
FIG. 4 shows plots of action potential recordings illustrating selective block of a slow conducting axon projecting from a small-diameter soma (B43) from *Aplysia californica,* while the action potential traveling down a fast conducting axon projecting from a large-diameter soma (B3) is unaffected;
FIG. 5 shows plots illustrating the selective block of slower-conducting compound action potential (CAP) components in the *Aplysia californica* plural-abdominal connective;

FIG. 6 shows plots illustrating the selective block of slower-conducting compound action potential (CAP) components in the *Suncus murinus* vagus nerve;

FIG. 7 shows a plot including traces of the temperature increase using parameters inhibiting axons in *Suncus murinus;* and

FIG. 8 is an illustration of an experimental setup for bath heating inhibition.

**Detailed Description**

**I. Definitions**

Unless otherwise defined, all technical terms used herein have the same meaning as commonly understood by one of ordinary skill in the art to which the present disclosure pertains.

As used herein, the term "neuromodulation" refers to the alteration of conduction in one or more nerve fibers through targeted delivery of a nerve modulating therapy to specific target areas in a subject's body. The term "modulation" can be used interchangeably with "neuromodulation".

As used herein, the term "nerve modulating therapy" refers to one or more modifying agents that modulate nerve activity upon delivery to a target area of the nervous system. The modifying agent can be any agent that affects the outer surface of one or more nerve fibers to modulate conduction in the one or more nerve fibers (e.g., a heat signal, a pressure wave, an optogenetic manipulation, a pharmaceutical dosage, or the like). For example, the nerve modulating therapy can affect the ion channels on the outer surface of the axon.

As used herein, the term "target area" refers to an area of a subject selected to receive the nerve modulating therapy. The target area can include small diameter and large diameter axons.

As used herein, the term "nerve" refers to one or nerve more fibers that employ electrical and chemical signals to transmit information. A nerve can refer to either a component of the central nervous system or the peripheral nervous system. For example, in the peripheral nervous system a nerve can transmit motor, sensory, autonomic, and/or enteric information from one body part to another.

As used herein, the term "nerve fiber" can refer to an axon of a neuron. The axon can conduct action potentials at a certain conduction velocity. The conduction velocity scales with axon diameter, such that large diameter axons conduct at a faster rate than small diameter axons. As an example, small diameter axons can have a diameter less than or equal to 4 $\mu$m. In another example, small diameter axons can have a diameter less than or equal to 2 $\mu$m.

As used herein, the term "conduction" refers to the transmission of one or more action potentials along a nerve fiber.

As used herein, the term "inhibition" refers to interference, with or restraint of, conduction. In other words, inhibition can be used to impede the transmission of action potentials along a nerve fiber.

As used herein, the term "intensity" refers to a measurable amount of a property of a modifying agent.

As used herein, the term "threshold" refers to a maximum or minimum change in intensity resulting in a functional result. For example, the functional result can be enhancing conduction or inhibiting conduction.

As used herein, the term "heat signal" refers to any signal that generates heat and can cause a change in temperature of a target area upon exposure or application thereto. Examples of heat signals can include, but are not limited to, optical signals (such as infrared (IR) light signals), radio frequency (RF) signals, ultrasound (US) signals, electrical heating signals, and the like.

As used herein, the term "radiant exposure" refers to an amount of radiant energy received by a surface per unit area (expressed in J/cm$^2$/pulse). Equivalently, the radiant exposure can be expressed as the irradiance that reaches a surface area due to irradiance, maintained for a particular duration of time.

As used herein, the term "pharmaceutical dosage" can refer to an amount of a drug sufficient to affect an outer surface of one or more nerve fibers to modulate conduction in the one or more nerve fibers. The pharmaceutical can be, for example, an ion channel blocker. The ion channel block can block ion channels in a small-diameter nerve fiber at a smaller dosage than that required to block ion channels in a large-diameter nerve fiber. The pharmaceutical dosage can be delivered, for example, by injection to a site, a drug-delivery polymer, or the like.

As used herein, the term "optogenetic manipulation" can refer to the use of light to control cells in neural tissue that have been genetically modified to express light-sensitive ion channels.

As used herein, the term "pressure wave" can refer to a signal in which a propagated disturbance is a variation of pressure in a natural medium. The terms "pressure signal" and "pressure wave" are used interchangeably herein.

As used herein, the term "sufficient" refers to an amount adequate to satisfy a condition.

As used herein, the term "neurological disorder" can refer to a condition or disease characterized at least in part by conduction in one or more nerve fibers. The neurological disorder can be in the motor system, the sensory system, and/or the autonomic system. As an example, function of an organ can be changed by modulating a neurological control signal.

As used herein, the terms "subject" and "patient" can be used interchangeably and refer to any warm-blooded organism including, but not limited to, a human being, a pig, a rat, a mouse, a dog, a cat, a goat, a sheep, a horse, a monkey, an ape, a rabbit, a cow, etc.

## II. Overview

The present disclosure relates generally to neuromodulation. Generally, neuromodulation refers to the alteration of conduction in one or more nerve fibers through targeted delivery of a nerve modulating therapy to a specific target area in a subject's body. The target area can include large-diameter axons and small-diameter axons. Small-diameter axons can play critical roles in sensory, autonomic, and motor systems. Thus, controlling the activity of these small-diameter axons is a large area of interest in the medical community. However, such selectivity for small-diameter axons does not yet exist. Accordingly, the present disclosure relates, more specifically, to systems and methods that target small-diameter axons in a target area for neuromodulation. In other words, conduction of the small-diameter axons in the target area can be modulated to a greater degree than conduction of large-diameter axons in the target area.

A nerve modulating therapy can include the application of one or more modifying agents to the target area. The modifying agent can be any agent that affects the outer surface of one or more nerve fibers within the target area to modulate conduction in the one or more nerve fibers. For example, the modifying agents can include a heat signal, a pressure wave, an optogenetic manipulation, a pharmaceutical dosage, or the like. The nerve modulating therapy can be configured to target the small-diameter axons and applied to a target area of a subject to modulate conduction in one or more small-diameter axons within the target area to a greater degree than large-diameter axons within the target area.

## III. Systems

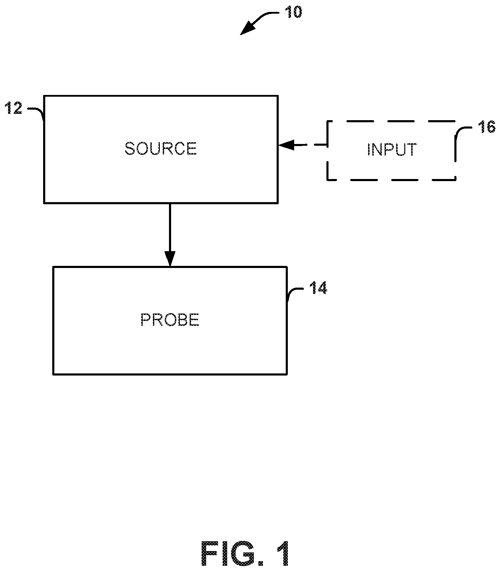

One aspect of the present disclosure can include a system 10 (FIG. 1) for configuring and applying a nerve modulating therapy to a target area of a subject's body. The system 10 can include a source 12 that can be configured to provide the nerve modulating therapy to a probe 14. The probe 14 can be configured for application of the nerve modulating therapy to the target area. The target area can be any area of the subject's body that includes nerve fibers of different sizes (e.g., small diameter nerve fibers and large diameter nerve fibers). Accordingly, the source 12 can configure the nerve modulating therapy to target the small diameter nerve fibers in the target area. As such, conduction in the small diameter nerve fibers can be modulated to a greater degree than the large diameter nerve fibers. The conduction in the small diameter fibers can be modulated so that the conduction is inhibited, but the modulation can also relate to enhancing the conduction in the small diameter fibers.

The nerve modulating therapy can include the application of one or more modifying agents to the target area to target the small diameter axons. The one or more modifying agents can include any agent that is applied to an outer surface the nerve fibers within the target area along the length of the nerve fibers to modulate conduction in the one or more nerve fibers. For example, the modifying agents can include a heat signal, a pressure signal, an optogenetic manipulation, a pharmaceutical dosage, or the like. The source 12 can configure the nerve modulating therapy to target the small diameter axons by selecting one or more modifying agents and selecting an intensity of the one or more modifying agents. The intensity required to target the small diameter fibers can be lower than the intensity required targeting the large diameter fibers.

The source 12 can configure the nerve modulating therapy with the modifying agent being a heat signal. The source 12 can be configured based on the specific type of heat signal being delivered. For example, the source 12 can be a laser source that provides infrared light, an ultrasound source that provides an ultrasound signal, a radio frequency source that provides a radio frequency signal, an electrical source that provides an electrical heating signal, or the like. When the heat signal is delivered at a proper intensity, the activation of voltage-dependent potassium channels can overwhelm the currents through ion channels on the surface of the nerve. The intensity can be represented by the radiant energy provided by the heat signal. A lower radiant energy is necessary to block small diameter fibers than would be necessary to block large diameter fibers.

The source 12 can configure the nerve modulating therapy with the modifying agent being a pressure wave. The source 12 can be configured based on the specific type of pressure signal being delivered. In one example, the pressure signal can be delivered by a laser source. In another example, the pressure signal can be delivered by an ultrasound source. Ultrasound can modulate nerve activity through pressure waves, which may open pressure-sensitive ion channels on the axon surface. The intensity of the pressure wave can be based on a magnitude of the pressure wave being applied.

The source 12 can configure the nerve modulating therapy with the modifying agent being an optogenetic manipulation. The source 12 can be a light source, like a laser, that provides a light signal to a genetically modified portion of the target area. The optogenetic manipulation can use light to affect ion channels. Accordingly, applications of the optogenetic manipulation should have similar intensity properties as the heat signal.

The source 12 can configure the nerve modulating therapy with the modifying agent being a pharmaceutical dosage. The source 12 can be an intravenous supply holder or an injectable holder, for example. In these instances, an amount of the pharmaceutical dosage can be applied primarily to a length of the surface of the nerve fibers in the target area. For example, the pharmaceutical dosage can include an ion channel blocker. With the pharmaceutical dosage, the intensity can be a concentration of the pharmaceutical dosage. A smaller concentration of the pharmaceutical dosage is necessary to target the small diameter nerve fibers in the target area than would be necessary to target the large diameter axons.

The source 12 can also configure the nerve modulating therapy to include a combination of a heat signal, pressure signal, optogenetic therapy, and/or a pharmaceutical dosage. For example, the nerve modulating therapy can include a heat signal and a pharmaceutical dosage. In this example, the pharmaceutical dosage enhances an effect of the heat signal. For example, the pharmaceutical dosage can reduce the amount of radiant energy that needs to be delivered by the heat signal to modulate the conduction in the small diameter fibers.

In some instances, the source 12 can receive an input 16 related to the configuration of the nerve modulating therapy. The input 16, for example, can indicate the modulating agent to apply, when to apply the modulating agent, and/or parameters of the application related to intensity. In some instances, the input 16 can come from a user (*e.g.,* the subject or a medical professional). In other instances, the input 16 can be at least partially based on closed loop feedback (*e.g.,* a recording of a property associated with the targeted small diameter fibers). The source 12 can adjust the intensity of the modulating agent that is applied by the probe 14 based at least in part on the input 16, for example.

The system 10 can also include the probe 14 that can be configured to deliver the configured nerve modulating therapy to the target area of the subject for application. In instances where the nerve modulating therapy includes a heat signal or pressure signal as the modifying agent, the probe 14 can be configured based on the type of heat signal or pressure signal. For example, in instances where the heat signal is an IR light signal, the probe 14 can include an optical waveguide configured to the target area, or the like. In another example, in instances where the heat signal is an US signal, the probe 14 can includes one or more ultrasonic transducers (*e.g.,* at least transmitters) arranged in a linear fashion, a curvilinear fashion, or a phased fashion. In instances where the nerve modulating therapy includes an optogenetic manipulation as the modifying agent, the probe 14 can be configured to deliver a light signal, such as including an optical waveguide configured to the target area, or the like. In instances where the nerve modulating therapy includes a pharmaceutical dosage as the modulating agent, the probe 14 can be an intravenous tube, a drug-releasing polymer construct configured to the target area, a needle, or the like.

### IV. Methods

Another aspect of the present disclosure can include a method 20 for targeting small-diameter axons for neuromodulation. The method 20 does not form part of the present invention and can be executed by hardware - for example, at least a portion of the system 10 shown in FIG. 1.

The method 20 is illustrated as a process flow diagram with flowchart illustrations. For purposes of simplicity, the method 20 is shown and described as being executed serially; however, it is to be understood and appreciated that the present disclosure is not limited by the illustrated order as some steps could occur in different orders and/or concurrently with other steps shown and described herein. Moreover, not all illustrated aspects may be required to implement the method 20. Additionally, one or more elements that implement the method 20, such as source 12 of FIG. 1, may include a non-transitory memory and one or more processors that can facilitate the configuration and generation of the heat signal.

At step 22, a nerve modulating therapy can be configured and generated (*e.g.,* by source 12). The nerve modulating therapy can be configured with one or more modifying agents, like a heat signal (*e.g.,* an infrared (IR) light signal, a radio frequency (RF) signal, an ultrasound (US) signal, an electrical heating signal, or any other type of signal that changes the temperature of a target area), an optogenetic modification, a pharmaceutical dosage, or the like. The nerve modulating therapy can also be configured with parameters related to the intensity delivered to the target area. The intensity to target small diameter axons is lower than the intensity required for larger diameter axons. For example, the threshold intensity to cause a change in conduction in a small diameter axon can be lower than the intensity necessary to cause a change in conduction in a large diameter axon. Parameters related to the intensity of the modifying agent (*e.g.,* wavelength, pulse width, radiant exposure, repetition rate, concentration, or the like) can be chosen to create an intensity greater than the threshold for modifying the conduction of a small diameter axon, but less than the intensity required to affect conduction in a large diameter axon.

At step 24, the nerve modulating therapy can be applied to the target area of a subject for a time (*e.g.,* by probe 14). The target area can be an area of a subject's body that includes a portion of the peripheral nervous system that includes axons with different diameters (*e.g.,* small diameter axons and large diameter axons). At step 26, small diameter axons in the target area can be targeted for neuromodulation. When exposed to the nerve modulating therapy, conduction of the small-diameter axons in the target area can be modulated to a greater degree than conduction of large-diameter axons in the target area

### V. Example Applications

A therapeutic treatment using the systems and methods described herein can target small diameter nerve fibers in a target area to modulate conduction to a greater degree than large diameter nerve fibers in the target area. Conduction can be modulated for 1 minute or more, in some instances. However, in other instances, conduction can be modulated for 10 minutes or more. In other instances, conduction can be modulated for 30 minutes or more. In still other instances, the conduction can be modulated for 9 hours or more. The modulation in conduction can refer to conditions of inhibition of conduction or to conditions of facilitation or excitation of conduction.

The therapeutic treatment can apply a heat signal, an optogenetic manipulation, a pharmaceutical dosage, or any combination thereof, to the target area at an intensity that targets the small diameter nerve fibers to modulate conduction. The ability to regulate neural activity with high spatial selectivity, while being selective by axon diameter size, has many possibilities. Nerves that can see a response from application of the heat signal include, but are not limited to, the nodose ganglion, the petrosal ganglion, the carotid body, the stellate ganglion, the dorsal root ganglia, the brainstem, the vagus nerve, the thoracic sympathetic nerve, the aortic depressor nerve, the hypoglossal nerve, and the phrenic nerve.

*Peripheral Nerve Control* - To control (excite or inhibit) peripheral nerves, the therapeutic treatment can exploit the ability of the nerve modulating therapy to be configured to inhibit conduction in neurons based on the size of a fiber's diameter. For example, the therapeutic treatment can apply the signal to a target area for inhibition of small diameter fibers in the target area. The therapeutic treatment can also benefit from the high spatial and temporal selectivity, rapidness, and reversibility of the signal. Applying nerve-modulating therapy configured with certain parameters (*e.g.,* wavelength, pulse width, radiant exposure, and/or repetition rate) to a target area that includes the small diameter conditions contributing to the neurological condition for a predetermined time of exposure can reverse at least some of the effects of the neurological condition. Examples of such neurological conditions that are at least partially affected by small-diameter fibers can include a myriad of conditions, including chronic nausea, vomiting, pain, hypertension, heart failure, obesity, and the like.

An infrared light source or waveguide can be implantable (*e.g.,* flexible polymer waveguide, vertical-cavity surface-emitting laser) and used to deliver infrared (IR) light to peripheral nerves. The intensity can be chosen so that the radiant exposure of the nerve is high enough to inhibit the small diameter axons, but low enough so as not to affect the conduction in the large diameter axons. The infrared laser can be used to alleviate symptoms and potentially cure diseases. For example, the infrared laser can be used on the vagus nerve to treat inflammatory diseases (*e.g.,* rheumatoid arthritis), pulmonary diseases (asthma, chronic pulmonary obstructive disease), and cardiovascular diseases (hypertension, atrial tachycardia, atrial fibrillation). Control of unmyelinated fibers in nerves, such as the sciatic nerve, can reduce or eliminate chronic pain.

*Inhibition of Unmyelinated C Fibers* - A nerve modulating therapy, such as a heat signal, can be configured to inhibit unmyelinated C fibers in the target area. Such C fibers carry sensory and pain information, which can be inhibited by a heat signal. A probe can be placed proximal to the target area to deliver the heat signal to the target area. Upon delivery of the heat signal, the target area can undergo a radiant exposure sufficient to inhibit the C fibers, but allow nerve fibers with larger diameters to continue conducting as normal. The probe can include, as one example, fiber optics implanted within a cuff electrode configured to surround a nerve to provide multiple points for delivery of the heat signal around the nerve's circumference. As another example, the probe can be fully implantable and consist of VCSELs or other light sources arranged circumferentially and/or longitudinally around the nerve and powered externally using inductive coils. The heat signal has advantages including one or more of the high spatial and temporal selectivity, rapidness, and reversibility provided by the heat signal. For example, the heat signal can be configured with parameters (*e.g.,* wavelength, pulse width, radiant exposure, repetition rate, or the like. For example, the heat signal can be configured with an intensity greater than the inhibition threshold) to selectively inhibit conduction in axons of a certain size and/or within a different spatial portion of the target area.

The heat signal can be any signal that changes the temperature of the target area. For example, the change in temperature can be between 2 and 14 degrees Celsius. However, the temperature change may be less than 2 degrees Celsius or greater than 14 degrees Celsius. The inhibition threshold, in some instances, can be based on the radiant energy. For example, the inhibition threshold can be a radiant exposure of $0.115 \text{ J/cm}^2\text{/pulse}$ or less. As another example, the inhibition threshold can be a radiant exposure of $0.015 \text{ J/cm}^2\text{/pulse}$ or less. The heat signal can also be configured with a certain exposure time. In some instances, the exposure time can be a total time the heat signal is applied. For example, the total time can be less than or equal to 1 minute, less than or equal to 5 minutes, less than or equal to 10 minutes, or the like. In other instances, the exposure time can be the time that a pulse of the heat signal is applied to the target area. For example, the pulses can be applied for less than or equal to 10 seconds, less than or equal to 15 seconds, less than or equal to 30 seconds, less than or equal to a minute, or the like.

*Inhibition aided by a Pharmaceutical Dosage* - Heat, at a certain radiant exposure, can be applied to a target area to inhibit conduction in a small diameter nerve fiber. The addition of a pharmaceutical dosage (*e.g.,* an ion channel blocker) can reduce the radiant exposure required to inhibit small-diameter axons. The use of a pharmaceutical dosage in combination with the heat signal can increase the safety and utility of inhibition of conduction in a small diameter nerve fiber. Application of the pharmaceutical dosage alone can also contribute to inhibition.

## VI. <u>Mathematical Analysis</u>

The following mathematical analysis demonstrates the diameter scaling of effects along the length of axons. For an equivalent axon length, the effect of thermal block on an axon scales with the square root of axonal diameter. For example, a large diameter axon may have a diameter four times the diameter of a small diameter axon of equivalent length. Applying the same nerve modulating therapy to the large diameter axon and the small diameter axon, the small diameter axon would exhibit a greater effect. This is true for other types of modifying agents that act along the length of axons (*e.g.,* pressure signal, optogenetic manipulation, pharmaceutical dosage, or the like).

This is exemplified by the following proof of a more general theorem - for any two axons of different diameter, if their physical properties along the length of the axon are identical, when position is scaled by the square root of diameter, then their behavior will be identical in this scaled coordinate system. To do this, a somewhat more general version of the cable equation is derived, which does not assume that the electrical properties of the cable, such as capacitance and conductance, are constant in space and time. The dependence on diameter is made explicit. Finally, this equation is rewritten in a new coordinate system (where length is scaled by the square root of diameter), and shows that diameter disappears from the equation in the new coordinate system.

Assume the axon is a cylinder of fixed diameter *b*. Let Cm be the membrane capacitance per unit area (which may vary over space or time). Then the capacitance per unit length along the axon, *Cm,* is

$$c_m = \pi b C_m$$

.

Similarly, let *Gk* be the conductance per unit area of the k-th current. The conductance per length of the current is then

$$g_k = \pi b G_k$$

.

Let *Pa* be the resistivity of the axoplasm; then the resistivity per unit length is

$$r_a = \frac{4\rho_a}{\pi b^2}$$

.

A more general form of the standard cable equation is derived that does not assume that the capacitance, conductance, and resistivity are fixed, so that they may vary over time and space. We first use Ohm's law to write the voltage drop along the axon:

$$\frac{\partial V}{\partial x} = -I_a r_a$$

,

where x is the position along the length of the axon and *Ia* is the current flowing along the axon in the direction of increasing *x*. Conservation of charge is then used to note that the change in current along the axon is equal to the net current flowing into the axon, or

$$\frac{\partial I_a}{\partial x} = i_m$$,

where $i_m$ is the net current flowing across the membrane per unit length.

To combine these two equations, $\frac{\partial V}{\partial x}$ is differentiated, yielding

$$\frac{\partial^2 V}{\partial x^2} = -\frac{\partial I_a}{\partial x} r_a - I_a \frac{\partial r_a}{\partial x}.$$

$\frac{\partial V}{\partial x} = -I_a r_a$ is used to substitute for $I_a$, producing

$$\frac{\partial^2 V}{\partial x^2} = -\frac{\partial I_a}{\partial x} r_a + \frac{1}{r_a} \frac{\partial V}{\partial x} \frac{\partial r_a}{\partial x}.$$

Solving for $\frac{\partial I_a}{\partial x}$,

$$\frac{\partial I_a}{\partial x} = -\frac{1}{r_a} \frac{\partial^2 V}{\partial x^2} + \frac{1}{r_a^2} \frac{\partial V}{\partial x} \frac{\partial r_a}{\partial x}.$$

Combining this with $\frac{\partial I_a}{\partial x} = i_m$ gives

$$i_m = -\frac{1}{r_a} \frac{\partial^2 V}{\partial x^2} + \frac{1}{r_a^2} \frac{\partial V}{\partial x} \frac{\partial r_a}{\partial x}.$$

The components of the membrane current are shown below. The definition of capacitance is used to write the charge per unit length stored on the membrane as

$$q_m = c_m V$$,

where V is the membrane potential at the given axial slice of the axon. Then $qm$ is differentiated to get the capacitive current per unit length flowing into the axon,

$$i_c = -\frac{\partial q_m}{\partial t} = -\frac{\partial c_m}{\partial t}V - c_m\frac{\partial V}{\partial t},$$

where $t$ is time. Note that the negative sign was introduced to preserve the direction of the current so that charging the membrane capacitance with a positive potential generates an outward (negative) flow. Ohm's law can be used to write the current per unit length flowing into the axon through channel $k$,

$$i_k = (E_k - V)g_k,$$

where $E_k$ is the reversal potential of channel $k$. The total membrane current is just the sum of the capacitive and channel currents, or

$$i_m = i_c + \sum_k i_k = -\frac{\partial c_m}{\partial t}V - c_m\frac{\partial V}{\partial t} + \sum_k(E_k - V)g_k.$$

Combining this equation with $i_m = -\frac{1}{r_a}\frac{\partial^2 V}{\partial x^2} + \frac{1}{r_a^2}\frac{\partial V}{\partial x}\frac{\partial r_a}{\partial x}$ and multiplying by -1 yields the desired more general version of the standard cable equation:

$$\frac{1}{r_a}\frac{\partial^2 V}{\partial x^2} - \frac{1}{r_a^2}\frac{\partial V}{\partial x}\frac{\partial r_a}{\partial x} = \frac{\partial c_m}{\partial t}V + c_m\frac{\partial V}{\partial t} + \sum_k(V-E_k)g_k.$$

To understand the effects of diameter, if the axon diameter is assumed only to enter the equation through its effects on $c_m$, $g_k$, and $r_{a''}$, their definitions can be used to make the dependence on diameter explicit in the more general version of the standard cable equation,

$$\left(\frac{4\rho_a}{\pi b^2}\right)^{-1}\frac{\partial^2 V}{\partial x^2} - \left(\frac{4\rho_a}{\pi b^2}\right)^{-2}\frac{\partial V}{\partial x}\left(\frac{4}{\pi b^2}\frac{\partial \rho_a}{\partial x}\right) = \left(\pi b\frac{\partial C_m}{\partial t}\right)V + (\pi b C_m)\frac{\partial V}{\partial t} + \sum_k(V-E_k)(\pi b G_k).$$

Dividing through by $\pi b$ and grouping $b$ with the partial derivatives,

$$\frac{1}{4\rho_a}\left(b\frac{\partial^2 V}{\partial x^2}\right) - \frac{1}{4\rho_a^2}\left(\sqrt{b}\frac{\partial V}{\partial x}\right)\left(\sqrt{b}\frac{\partial \rho_a}{\partial x}\right) = \frac{\partial C_m}{\partial t}V + C_m\frac{\partial V}{\partial t} + \sum_k(V-E_k)G_k.$$

A change of coordinates is performed in an attempt to isolate the effects of the diameter $b$. A new spatial coordinate $u$ is defined such that

$$x = \sqrt{b}\,u$$

Then

$$\frac{\partial x}{\partial u} = \sqrt{b}$$

and thus for an arbitrary smooth function $f$,

$$\frac{\partial f}{\partial u} = \frac{\partial x}{\partial u}\frac{\partial f}{\partial x} = \sqrt{b}\,\frac{\partial f}{\partial x}$$

and

$$\frac{\partial^2 f}{\partial u^2} = \frac{\partial^2 x}{\partial u^2}\frac{\partial f}{\partial x} + \frac{\partial x}{\partial u}\left(\frac{\partial x}{\partial u}\frac{\partial^2 f}{\partial x^2}\right) = 0\frac{\partial f}{\partial x} + \sqrt{b}\left(\sqrt{b}\,\frac{\partial^2 f}{\partial x^2}\right) = b\,\frac{\partial^2 f}{\partial x^2}$$

Applying these identities to

$$\frac{1}{4\rho_a}\left(b\,\frac{\partial^2 V}{\partial x^2}\right) - \frac{1}{4\rho_a^2}\left(\sqrt{b}\,\frac{\partial V}{\partial x}\right)\left(\sqrt{b}\,\frac{\partial \rho_a}{\partial x}\right) = \frac{\partial C_m}{\partial t}V + C_m\frac{\partial V}{\partial t} + \sum_k (V - E_k)G_k ,$$

the equation becomes

$$\frac{1}{4\rho_a}\frac{\partial^2 V}{\partial u^2} - \frac{1}{4\rho_a^2}\frac{\partial V}{\partial u}\frac{\partial \rho_a}{\partial u} = \frac{\partial C_m}{\partial t}V + C_m\frac{\partial V}{\partial t} + \sum_k (V - E_k)G_k .$$

This final version of the cable equation proves the hypothesis. To see this, note that the final version of the cable equation has no dependence on the diameter $b$, only $u$. Thus, if two axons have the same membrane capacitance, conductance per unit area and axonal resistivity when expressed in u-coordinates, then they have identical behavior in u-coordinates. As a specific example, if an axon with diameter $b$ can be blocked by a high-temperature region of a minimum length $l$, then an axon of diameter c with otherwise identical properties will be blocked by a high-temperature region of minimum length $l\sqrt{c}/\sqrt{b}$ . . The power and generality of this mathematical model allow the assertion that any particular biophysical model of an axon that satisfies the assumptions of the cable equation will show the same scaling law. Since the model is completely general, it makes predictions about any modality that affects neurons along their surface

### VII. Experimental

The following example is shown for the purpose of illustration only and is not intended to limit the scope of the invention which is defined by the appended claims. This experiment demonstrates selective inhibition of small-diameter axons using a heat signal provided by infrared (IR) light (*e.g.,* wavelength of 1464nm or 1860 nm, 200 Hz frequency, 200 $\mu$s pulse duration). IR inhibition may be due to an increase in baseline temperature. By changing laser parameters (*e.g.,* wavelength, pulse width, radiant exposure, repetition rate, and the like), one can selectively and preferentially inhibit small diameter neurons. Additionally, this experiment strongly supports the mathematical analysis and suggests that any modifying agent applied primarily to the axonal surface would preferentially affect small-diameter axons at a lower intensity.

**Methods**

*Aplysia Model*

The marine mollusks *Aplysia californica* (Marinus Scientific, Long Beach, CA) were maintained in an aerated aquarium circulating artificial seawater (Instant Ocean, Aquarium Systems, Mentor, OH) kept at 16-17°C, with a 12-hour dark/ 12-hour light cycle. The aquarium contained macroalgae and organisms simulating the ecosystem in which marine slugs normally live. The animals were fed a diet of dried seaweed every other day. Inter-bite intervals (3 - 5s) in response to a seaweed strip indicating normal health were used for selecting animals.

*Shrew Model*

Experimentally naive adult male musk shrews were used *(Suncus murinus; >35* days of age). The male shrew was used, as they were often larger in size than females and generally had larger and thicker vagus nerves, making the surgeries and bundle dissections easier and more likely to succeed. Musk shrews were obtained from a breeding colony at the University of Pittsburgh Cancer Institute and were descendants from animals acquired from the Chinese University of Hong Kong, a Taiwanese strain. Animals were singly housed in clear plastic cages, with a filtered air supply, under a 12-hour standard light cycle (lights on at 07:00 AM), in a temperature (~23°C) and humidity (~40%) controlled environment. Food and drinking water were freely available, but food was removed 2 hours before euthanasia and removal of the vagus nerve. Food consisted of a mixture of 75% Purina Cat Chow Complete Formula and 25% Complete Gro-Fur mink food pellets.

Experiments were approved by the University of Pittsburgh Institutional Animal Care and Use Committee and conducted in compliance with USDA guidelines. Animals were housed in an Association for Assessment and Accreditation of Laboratory Animal Care international-accredited animal care facility.

*Laser*

A tunable diode laser (Capella; Lockheed-Martin Aculight, Bothell, WA) with a wavelength $\lambda$ = 1860 nm was used and block was induced by applying 200 $\mu$s pulses at 200 Hz. The IR laser was coupled into an optical fiber whose diameter corresponded to the cross-section of the target nerve. For all *Aplysia* experiments, the diode laser was coupled to a 600 $\mu$m multimode optical fiber (P600-2-VIS-N1 R, Ocean Optics, Dunedin, FL) positioned at a 90° angle over the nerve using a micromanipulator. The optical fiber gently touched the nerve sheath. Shrew experiments were similar to those in *Aplysia,* except that a 400 $\mu$m optical fiber was used.

At the end of each experiment, the pulse energies at which block was obtained were measured using a pyroelectric energy meter (PESOBB, Ophir-Spiricon, North Logan, UT). From these measurements, the radiant exposure ($J/cm^2$/pulse) effective at producing optical block could be established by dividing the individual pulse energies by the laser spot size. Instead of making assumptions to determine the laser spot size at the axons, the radiant exposures at the fiber tip was reported.

A thermal camera (FLI R A325sc, Wilsonville, OR) was used along with the ResearchIR software to assess laser-induced temperature changes to the tissue as reported in Wang, Y.T., Rollins, A.M., and Jenkins, M.W. "Infrared inhibition of embryonic hearts". J. Biomed. Opt. 21, 60505 (2016).

Preliminary tests comparing temperature rise in nerves in Krebs solution and water alone showed no discernible differences so water was used to simplify the experiments. Briefly, one rounded edge of a Petri dish was cut off and replaced with a flat cover slip. The Petri dish was then filled with water. A 400 $\mu$m (shrew experiments) or 600 $\mu$m (*Aplysia* experiments) optical fiber was placed just barely touching the surface of the water and with the cross-section bisected by the glass-water interface. By assuming an axially symmetric temperature distribution and taking into account that glass has a high thermal conductivity and a low specific heat compared to water, thermal imaging at the cover slip surface provided an accurate measure of temperature distribution in depth through the middle of the heated region.

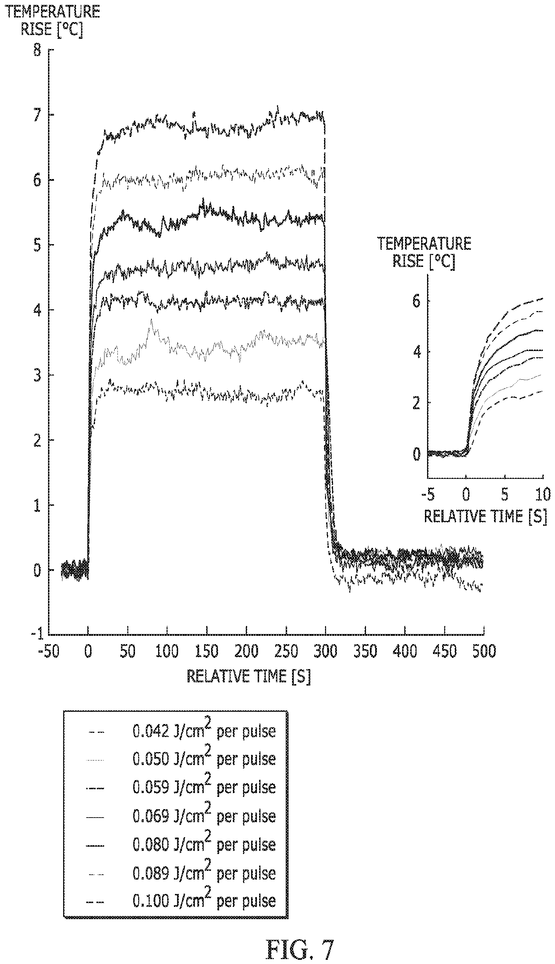

A range of laser energies that corresponded to values used in experiments was tested. For each laser level, recordings were taken for 700 seconds. The laser was applied for a 300-second window between 101-400 seconds, which provided time for the temperature to reach a steady state and return to baseline after turning the laser off (FIG. 7). To determine the actual temperature threshold for inhibition within the nerve, the time point on the temperature profile for a specific radiant exposure corresponding to how long it took to achieve block was used. A piecewise cubic Hermite interpolating polynomial (PCHIP) interpolation was employed when the measured radiant exposure fell between the measured traces.

*Intracellular identified cell and axon experiments*

*Aplysia californica* (a total of N = 7 animals, 8 nerves) weighing 250-350g were used for these experiments. Animals were anesthetized with an injection of MgCh (-50% of body weight) prior to dissection. Once anesthetized, the buccal ganglion and associated nerve, buccal nerve 2 (BN2), were dissected out of the animal. The nerve was cut distally prior to the trifurcation into separate branches. After pinning the buccal ganglion to the dish containing Sylgard (Dow Corning, Auburn, MI), the protective sheath of the buccal ganglion was removed to allow access to the nerve cell somata with intracellular glass electrodes. The nerve and the ganglion were immersed in a mixture of high-divalent cation *Aplysia* saline (270 mM NaCl, 6 mM KCl, 120 mM $MgCl_2$, 33 mM $MgSO_4$, 30 mM $CaCl_2$, 10 mM glucose, and 10 mM 3-(N-morpholino) propanesulfonic acid, pH 7.5).

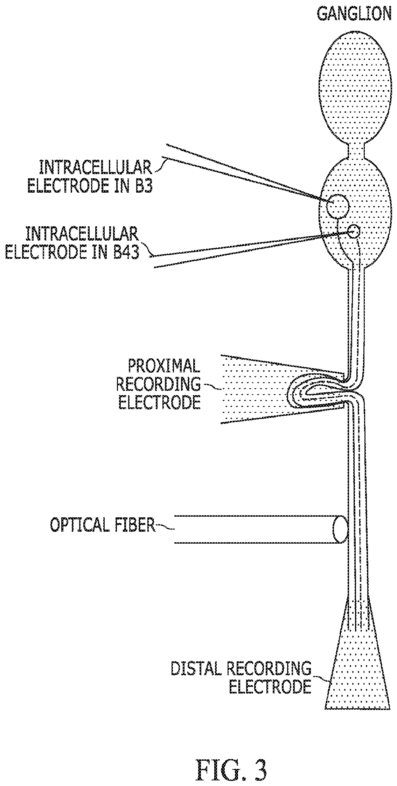

Intracellular glass electrodes were used to impale identified neurons B3 and B43 to record and control their voltage [FIG. 3]. The electrodes were pulled from thin-walled filament capillary glass (1.0 mm outer diameter, 0.75 mm inner diameter, A-M Systems, Sequim, WA) using a Flaming/Brown micropipette puller (model P-80/PC, Sutter Instruments, Novato, CA) and had an inner diameter ranging from 3-6 $\mu$m. Electrodes were backfilled with 3 M potassium acetate before use. The bridge was balanced for stimulation and recording. The identified cells were stimulated at a frequency of 2Hz. Intracellular signals were amplified using a DC-coupled amplifier (model 1600, A-M Systems, Sequim, WA).

To record action potentials travelling down the length of the nerve, extracellular suction electrodes were positioned along the length of BN2. The electrodes were made by pulling polyethylene tubing (Becton Dickinson, Franklin Lakes, NJ; #427421; outer diameter 1.27 mm, inner diameter 0.86 mm) placed over a flame to obtain an electrode whose diameter matched the nerve. Prior to suctioning the nerve, each extracellular electrode was filled with high-divalent cation *Aplysia* saline. Two extracellular electrodes were placed on BN2: one *en passant* electrode mid-way along the length of the nerve, and one suction electrode at the cut end of the nerve. An Ag/AgCl-coated wire was inserted in the recording electrodes. Recordings from extracellular electrodes were amplified using an AC-coupled differential amplifier (model 1700, A-M Systems, Sequoia, WA) and filtered using a 500 Hz low-pass and a 300 Hz high pass filter. Data were digitized and recorded for analysis using AxoGraph X.

Thresholds for reliably inducing action potentials were determined individually for the larger-diameter neuron (B3) and axon, and the smaller-diameter neuron (B43) and axon. Conduction velocities were determined for each neuron and axon (N = 6 for B3, N = 3 for B43). Radiant exposure block thresholds were then established for the small diameter B43 axon and the large-diameter B3 axon (N = 5 for pairs of axons). The neurons were electrically stimulated after infrared light application to assess nerve health and IR block reversibility.

*Aplysia* whole nerve *in vitro* experiments

To separate the axonal sub-populations with different conduction velocities, a longer nerve (the *Aplysia* pleural-abdominal connective) was used. Larger animals weighing 350-410g were used because they have longer nerves (N = 7 animals). The ganglia on either end of the nerve were dissected away. The nerve was placed in a Sylgard recording dish containing *Aplysia* saline (460 mM NaCl, 10 mM KCl, 22 mM $MgCl_2$, 33 mM $MgSO_4$, 10 mM $CaCl_2$, 10 mM glucose, and 10 mM 3-(N-morpholino) propanesulfonic acid, pH 7.5), and its sheath was pinned down.

To stimulate the nerve, a monopolar extracellular suction electrode was placed at one cut end of the nerve. The stimulation electrode was grounded using a return electrode placed in the dish's saline. The nerve was stimulated at a frequency of 2 Hz. A bipolar extracellular recording electrode composed of an *en passant* and a suction electrode was placed at the other end of the nerve. The bipolar recording electrode reduced recording noise. Electrodes were filled with *Aplysia* saline before suctioning the nerve to preserve its viability. Signals were amplified using the extracellular amplifier described above, and the nerve CAP was digitized and recorded using AxoGraph X.

Thresholds for reliably inducing all CAP components were determined. It was observed that if currents significantly higher than threshold were used, additional components to the CAP were recruited that were of intermediate velocity and highly resistant to thermal block. To prevent this from happening, the stimulation amplitudes were kept just above threshold. Conduction velocities were determined for the different CAP sub-components (N = 3). Radiant exposure block thresholds were then established for the slower, smaller-diameter sub-components (N =7). The nerve was electrically stimulated after infrared light application to assess nerve health and IR block reversibility.

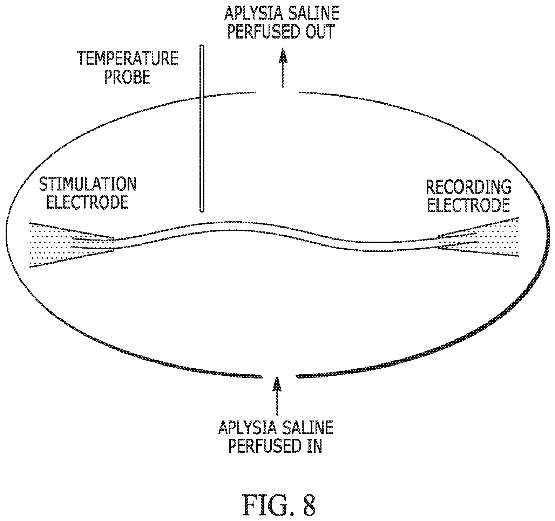

The *in vitro* bath heating experiments (N = 4) used a preparation as shown in FIG. 8. A stimulation suction electrode was placed on one end of the nerve and a monopolar recording suction electrode was placed on the other end of the nerve. The nerve was stimulated at a frequency of 2 Hz, and the signal was amplified using an external amplifier.

Current amplitude threshold for reliable stimulation of all CAP components was determined at room temperature (21.5-24.5 °C). *Aplysia* saline, warmed using a water bath (model EX-211, NESLAB Instruments, Newington, NH) and an in-line heating system (model TC-344C [temperature controller], SH-278 [in-line heater], Warner Instruments, Hamden, CT), was perfused using a peristaltic pump (model MasterFlex 7524-10, Cole Parme, Vernon Hills, IL) through the dish. Its temperature was monitored using a temperature probe (model SDL200, Extech Instruments, Nashua, NH) and digitized using (model SMCJ-K, OMEGA Engineering, Norwalk, CT). The bath temperatures tested ranged from room temperature to 39.8 ± 0.4 °C. After reaching 39.8 ± 0.4 °C, cold saline was added to the bath to return it to room temperature and assess the nerve's health. The nerve was continuously stimulated throughout the experiment to monitor its ability to conduct at the varying temperatures.

*Shrew whole nerve in vitro experiments*

Animals (N = 3 nerves from 3 separate animals) were euthanized by exposure to $CO_2$ until lack of respiration, followed by cervical dislocation. The thoracic cavity was opened to reveal the heart and an incision was made in the cardiac apex to drain the blood. This method was used to reduce bleeding in the neck when excising the vagus. A midline incision was then made in the ventral surface of the neck, including a cut through the clavicle bones to expose the left vagus trunk, which exposed the segment of the vagus from above the heart to the nodose ganglion. This cervical plus thoracic vagal segment was removed and placed in cold Krebs solution (5 to 7°C). The time from euthanasia to placing the nerve in Krebs solution was less than 5 min. The nerve was then further dissected in a dish containing Krebs (which was continually oxygenated) to remove excess connective tissue before placement in a three-compartment chamber for electrophysiology recordings. Krebs solution was perfused through the middle compartment at a rate of 5.1 ml/min.

In the nerve stimulation compartment, the nerve was pinned at the end and draped across two platinum-iridium hook electrodes (separated by 0.5 mm). The nerve and electrodes were encased in Kwik-cast silicone (WPI, Sarasota, FL) and the compartment was filled with mineral oil. Nerve stimulation was produced by applying biphasic pulses through the stimulation electrodes (0.5 ms duration; 0.5 s inter-pulse interval; 0.04 to 0.11 mA, depending on which current level would allow for reliable stimulation of all axonal sub-populations. Once selected, the current level was kept constant throughout the experiment).

The recording compartment was also filled with mineral oil, and the nerve was positioned across a reference electrode. When recording from the whole vagus, the noise obscured the activity of slower-conducting fibers. For that reason, we dissected out small bundles from the cervical vagus from which to record. In each experiment, a nerve bundle was dissected from the nerve trunk and wrapped around a recording electrode. Signals were acquired at an amplification of 5,000 using a differential AC amplifier (P511, Grass Instruments, Natus Medical Inc., Pleasanton, CA; 100 and 1,000 Hz cutoffs) and recorded to computer (Spike 2, CED, Cambridge, England).

The experimental design of the shrew *in vitro* experiments closely followed the experimental design used for the *Aplysia* whole nerve *in vitro* experiments. The only difference is that each experiment was repeated three times in each animal.

For transmission electron microscopy, nerves were harvested and immersion fixed (2.5% glutaraldehyde, 2% paraformaldehyde in PBS) overnight at 4°C. Following fixation, tissue was washed 3x in PBS then post-fixed in aqueous 1 % $OsO_4$, 1 % $K_3Fe(CN)_6$ for 1 hour. Following 3 PBS washes, the tissue was dehydrated through a graded series of 30-100% ethanol, 100% propylene oxide, and then infiltrated in a 1:1 mixture of propylene oxide: Polybed 812 epoxy resin (Polysciences, Warrington, PA) for 1 hour. After several changes of 100% resin over 24 hours, the pellet was embedded in molds, cured at 37°C overnight, followed by an additional hardening at 65°C for two more days. Semi-thin (300 nm) cross-sections were heated onto glass slides, stained with 1 % toluidine blue and imaged using light microscopy. Ultrathin (60 nm) cross sections of nerve were collected on copper grids, stained with 1 % uranyl acetate for 10 minutes, followed by 1 % lead citrate for 7 minutes. Sections were imaged using a JEOL JEM 1400 transmission electron microscope (JEOL, Peabody, MA) at 80 kV fitted with a side mount AMT 2k digital camera (Advanced Microscopy Techniques, Danvers, MA).

*Data and Statistical Analysis*

For single cell experiments, a Mann Whitney test determined whether conduction velocities in axons projecting from B3 and B43 were statistically different. A paired t-test determined whether threshold radiant exposure levels for inhibiting action potentials in B3 and B43 were statistically different.

For whole nerve experiments, data were digitally filtered using a 50 Hz 4th order high-pass Butterworth filter, and a 1000 Hz 4th order low-pass Butterworth filter. By identifying the onset of the artifact, each stimulation trial was extracted. Because waveform shapes can be changed both by shift in the underlying action potentials with temperature as well as by complete block, stable regions within the CAP were identified by finding the areas of lowest variance across all stimulation traces. Within each of these stable regions, corresponding to different conduction velocities, the rectified area under the curve (RAUC) was calculated. Medians were plotted, surrounded by dashed lines representing the first and third quartiles of the data for successive stimulation groups.

Results were converted to binary categorical data (1 - no significant decrease of RAUC, 0 - RAUC was reduced to less than 1 /e compared to traces recorded before the IR laser was on). The same experiment was repeated three times on three different preparations, and the results were analyzed using the Cochran Mantel-Haenszel test to remove any possible influences from biological variability among the three experiments. For the *Aplysia* data, the standard chi-squared test was used. When multiple comparisons were tested in the same experimental set, the Bonferroni correction was applied to control the overall Type I error. To reach statistical significance, the overall *p* value was set at 0.05 before the Bonferroni correction.

**Results**

The following results demonstrate the selective inhibition of small-diameter axons using IR light.

*Preferential Inhibition of Small Diameter Fibers by IR Light in Invertebrates*

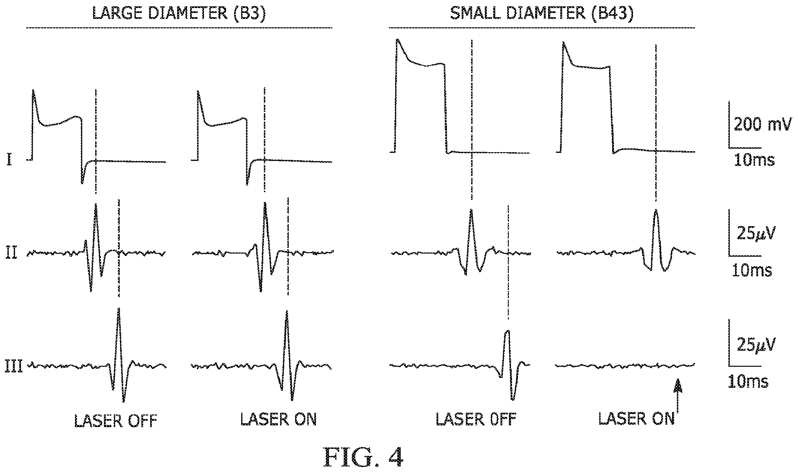

In an invertebrate preparation *(Aplysia californica)*, recordings were taken from the somata of two identified neurons, B3 and B43. The mean conduction velocity of B3 was found to be 221 % higher than that of B43 [p = 0.0271, Mann Whitney test]. It was observed that a lower radiant exposure inhibited B43 compared to B3 ($0.097\pm0.026$ $J/cm^2$/pulse versus $0.126\pm0.030$ $J/cm^2$/pulse) [FIG. 4; p = 0.0091]. Higher radiant exposures inhibited both axons. These effects were rapidly reversible (within 0.5 s).

*Selective Inhibition of Populations of Small-Diameter Fibers by IR Light in Invertebrates*

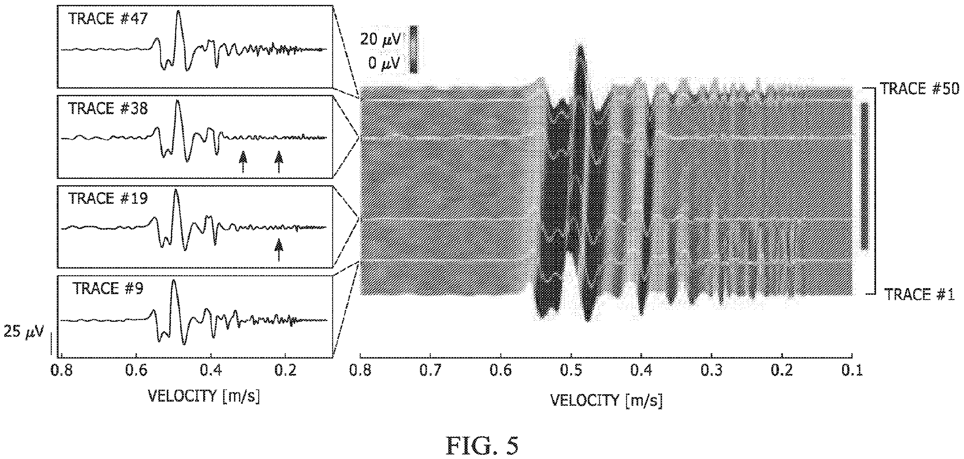

The pleural-abdominal connective of *Aplysia,* which contained only unmyelinated axons whose most common axonal diameter ranges from 0.8-3 $\mu$m was used. Electrical stimulation of the nerve generated a compound action potential (CAP), which included fast-conducting (large-diameter) and slow-conducting (small-diameter) axons. These components separate from one another over the length of the nerve. Within 11 seconds of a IR laser being turned on at a radiant exposure of 0.140 J/cm$^2$/pulse, the slower components (0.43-0.18 m/s) of the CAP were blocked [FIG. 5, trace 38 compared to trace 9]. Once the IR laser was turned off, all components of the CAP returned [FIG. 5, trace 47]. Over the 50 traces, the process of inhibition selectively affected the slowest components [FIG. 5, contour plot]. To quantify the changes, the CAP was divided into regions at points of low variability, and the rectified area under the curve (RAUC) was measured for each region. Experiments were conducted on 3 animals. Using chi-squared tests, slow-velocity components showed statistically significant reductions in RAUC when compared to the fast-velocity components in all three preparations. The average radiant exposure to block the smaller components was 0.110$\pm$0.027 J/cm$^2$/pulse, and the measured temperature increase was 9.7$\pm$3.7°C.

*Selective Inhibition of Small-Diameter Axons is Due to a Thermal Effect*

To demonstrate that the selective inhibition of axonal subpopulations is due to a thermal effect, the *Aplysia* pleural-abdominal connective was placed in a saline bath while controlling temperatures. As temperature increased, the slow-conducting components of the compound action potential were preferentially blocked. As the bath temperature increased to still higher values, all components of the compound action potential eventually were inhibited.

*Preferential Inhibition of Small-Diameter Axons by IR Light in Vertebrates*

The vagus of a mammal, the musk shrew, *Suncus murinus,* was studied to test whether populations of small diameter axons in vertebrates can be preferentially inhibited, even though they have different components of ion channels than those in *Aplysia.* The vagus is a mixed nerve, containing both myelinated and unmyelinated axons. To measure changes in slow-conducting fibers, the fiber numbers were reduced by dissecting a small bundle of axons from the cervical end of an *in vitro* vagus preparation. The CAP was induced by electrical shock at the upper thoracic end and was recorded from the cervical bundle. The IR laser was also applied to the cervical vagus between stimulating and recording electrodes.

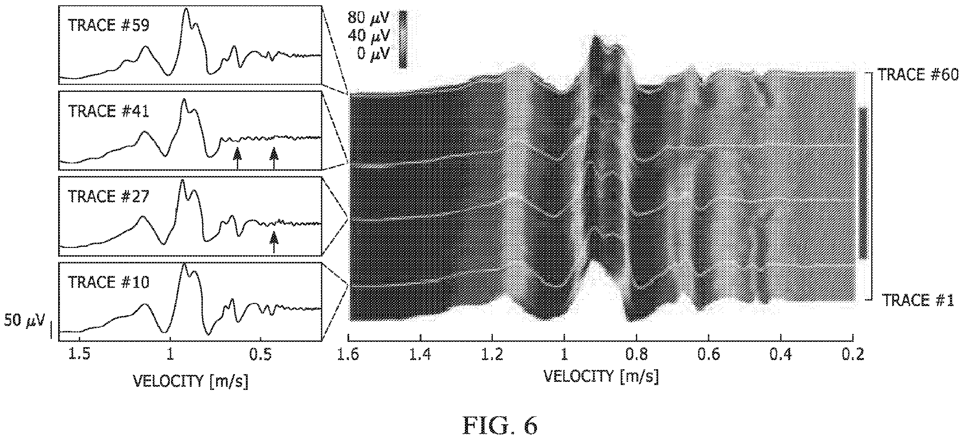

Within 14 seconds after the laser was turned on at a radiant exposure of 0.064 J/cm$^2$/pulse, the slowest and intermediate components (0.68-0.35 m/s) of the CAP were blocked [FIG. 6, trace 41 compared to trace 10]. Once the laser was turned off, all components of the CAP returned [FIG. 6, trace 59]. Over the 60 traces, the process of inhibition selectively affected the slowest components [FIG. 6, contour plot]. To quantify the changes, the CAP was divided into regions of low variability, and the RAUC was measured. Each experiment was repeated 3 times/animal and in 3 different animals. Using Cochran-Mantel-Haenszel tests, slow-velocity components showed statistically significant reductions when compared to fast-velocity components in all preparations. The average radiant exposure to block the smaller components was 0.050$\pm$0.012 J/cm$^2$/pulse and the measured temperature increase was 2.9$\pm$0.8°C.

To demonstrate the presence of unmyelinated axons in the bundle, transmission electron microscopy was performed. Unmyelinated axons ranged from 0.5-2.0 $\mu$m in ferret diameter, whereas myelinated axons ranged from 1.5-15.0 $\mu$m.

From the above description, those skilled in the art will perceive improvements, changes and modifications. Such improvements, changes and modifications are within the skill of one in the art and are intended to be covered by the appended claims. The scope of the invention is defined by the appended claims.

**Claims**

1.  A system (10) comprising:

    an implantable probe (14) configured to apply a nerve modulating therapy to a target area of a subject to modulate conduction in one or more small diameter nerve axons to a greater degree than in one or more large diameter axons, wherein the target area includes the one or more small diameter nerve axons and the one or more large diameter nerve axons;
    a source (12), communicatively coupled to the implantable probe, configured to:

configure the nerve modulating therapy to modulate conduction in the one or more small diameter nerve axons by:

selecting a heat signal or an optogenetic modulation to provide the nerve modulating therapy, and selecting an intensity level of the heat signal or the optogenetic modulation provided by a given amount of radiant exposure to the target area that modulates conduction in ion channels on an outer surface of the one or more small diameter nerve axons while leaving ion channels on an outer surface of the one or more large diameter nerve axons unaffected, and

provide the nerve modulating therapy to the implantable probe wherein the implantable probe (14) is configured to apply the nerve modulating therapy to the target area of the subject for a time to modulate conduction in the one or more small diameter axons within the target area to a greater degree than large diameter axons within the target area,

wherein the source is configured to receive an input (16) based on a recording of a property associated with the one or more small diameter axons, and adjust the intensity level of the heat signal or the optogenetic modulation based on the input by adjusting the given radiant exposure of the target area.

2. The system of claim 1, wherein the selected nerve modulation therapy is the heat signal, and the heat signal is provided by a light signal, a laser source, or an ultrasound source.

3. The system of claim 1, wherein the nerve modulating therapy comprises the heat signal and a pharmaceutical dosage, wherein the pharmaceutical dosage enhances an effect of the heat signal.

4. The system of claim 1, wherein modulation of conduction in the one or more small diameter axons comprises inhibiting conduction in the one or more small diameter axons or exciting conduction in the one or more small diameter axons.

5. The system of claim 1, wherein the source is further configured to adjust the nerve modulating therapy to target other small diameter axons within a different spatial portion of the target area.

**Patentansprüche**

1. System (10), umfassend:

eine implantierbare Sonde (14), die konfiguriert ist, um eine Nervenmodulationstherapie auf einen Zielbereich eines Subjekts anzuwenden, um das Leiten in einem oder mehreren Nervenaxonen mit kleinem Durchmesser in einem größeren Maße zu modulieren als in einem oder mehreren Axonen mit großem Durchmesser, wobei der Zielbereich das eine oder die mehreren Nervenaxone mit kleinem Durchmesser und das eine oder die mehreren Nervenaxone mit großem Durchmesser einschließt;

eine Quelle (12), die kommunikativ mit der implantierbaren Sonde gekoppelt, die konfiguriert ist zum:

Konfigurieren der Nervenmodulationstherapie, um das Leiten in dem einen oder den mehreren Nervenaxonen mit kleinem Durchmesser durch Folgendes zu modulieren:

Auswählen eines Wärmesignals oder einer optogenetischen Modulation, um die Nervenmodulationstherapie bereitzustellen, und

Auswählen eines Intensitätsniveaus des Wärmesignals oder der optogenetischen Modulation, das durch einen gegebenen Umfang von Bestrahlung des Zielbereichs bereitgestellt ist, der das Leiten in Ionenkanälen auf einer Außenfläche des einen oder der mehreren Nervenaxone mit kleinem Durchmesser moduliert, während Ionenkanäle auf einer Außenfläche des einen oder der mehreren Nervenaxone mit großem Durchmesser unbeeinflusst bleiben, und

Bereitstellen der Nervenmodulationstherapie für die implantierbare Sonde, wobei die implantierbare Sonde (14) konfiguriert ist, um die Nervenmodulationstherapie auf den Zielbereich des Subjekts eine Zeit lang anzuwenden, um das Leiten in dem einen oder den mehreren Axonen mit kleinem Durchmesser in dem Zielbereich in einem größeren Maße zu modulieren als in Axonen mit großem Durchmesser in dem Zielbereich,

wobei die Quelle konfiguriert ist, um eine Eingabe (16) basierend auf einer Aufzeichnung einer Eigenschaft zu empfangen, die mit dem einen oder den mehreren Axonen mit kleinem Durchmesser in Zusammenhang steht, und das Intensitätsniveau des Wärmesignals oder der optogenetischen Modulation basierend auf der Eingabe durch Justieren der gegebenen Bestrahlung des Zielbereichs zu justieren.

**2.** System nach Anspruch 1, wobei die ausgewählte Nervenmodulationstherapie das Wärmesignal ist und das Wärmesignal durch ein Lichtsignal, eine Laserquelle oder eine Ultraschallquelle bereitgestellt wird.

**3.** System nach Anspruch 1, wobei die Nervenmodulationstherapie das Wärmesignal und eine pharmazeutische Dosis umfasst,
wobei die pharmazeutische Dosis eine Wirkung des Wärmesignals verstärkt.

**4.** System nach Anspruch 1, wobei die Modulation des Leitens in dem einen oder den mehreren Axonen mit kleinem Durchmesser das Hemmen des Leitens in dem einen oder den mehreren Axonen mit kleinem Durchmesser oder das Erregen des Leitens in dem einen oder den mehreren Axonen mit kleinem Durchmesser umfasst.

**5.** System nach Anspruch 1, wobei die Quelle ferner konfiguriert ist, um die Nervenmodulationstherapie zu justieren, um auf andere Axone mit kleinem Durchmesser in einem anderen räumlichen Abschnitt des Zielbereichs abzuzielen.

**Revendications**

**1.** Système (10) comprenant :

une sonde implantable (14) configurée pour administrer une thérapie de neuromodulation sur une zone cible d'un sujet pour moduler la conduction dans un ou plusieurs neuroaxones de petit diamètre selon un degré plus important que dans un ou plusieurs axones de grand diamètre, dans lequel la zone cible inclut les un ou plusieurs neuroaxones de petit diamètre et les un ou plusieurs neuroaxones de grand diamètre ;
une source (12), couplée en communication à la sonde implantable, configurée pour :
configurer la thérapie de neuromodulation pour moduler la conduction dans les un ou plusieurs neuroaxones de petit diamètre en :

sélectionnant un signal thermique ou une modulation optogénétique pour fournir la thérapie de neuromodulation, et en
sélectionnant un niveau d'intensité du signal thermique ou de la modulation optogénétique fourni au moyen d'une quantité donnée d'exposition au rayonnement sur la zone cible qui module la conduction dans les canaux ioniques sur une surface externe des un ou plusieurs neuroaxones de petit diamètre tout en laissant non affectés les canaux ioniques sur une surface externe des un ou plusieurs neuroaxones de grand diamètre, et pour
fournir la thérapie de neuromodulation à la sonde implantable, dans lequel la sonde implantable (14) est configurée pour administrer la thérapie de neuromodulation sur la zone cible du sujet pendant une durée pour moduler la conduction dans les un ou plusieurs axones de petit diamètre à l'intérieur de la zone cible selon un degré plus important que dans des axones de grand diamètre à l'intérieur de la zone cible ;
dans lequel la source est configurée pour recevoir une entrée (16) sur la base d'un enregistrement d'une propriété associée aux un ou plusieurs axones de petit diamètre, et pour régler le niveau d'intensité du signal thermique ou de la modulation optogénétique sur la base de l'entrée en réglant l'exposition au rayonnement donnée de la zone cible.

**2.** Système selon la revendication 1, dans lequel la thérapie de neuromodulation sélectionnée est le signal thermique, et le signal thermique est fourni par un signal lumineux, une source laser ou une source d'ultrasons.

**3.** Système selon la revendication 1, dans lequel la thérapie de neuromodulation comprend le signal thermique et un dosage pharmaceutique,
dans lequel le dosage pharmaceutique améliore un effet du signal thermique.

**4.** Système selon la revendication 1, dans lequel la modulation de la conduction dans les un ou plusieurs axones de petit diamètre comprend l'inhibition de la conduction dans les un ou plusieurs axones de petit diamètre ou l'excitation de la conduction dans les un ou plusieurs axones de petit diamètre.

**5.** Système selon la revendication 1, dans lequel la source est en outre configurée pour ajuster la thérapie de neuromodulation pour cibler d'autres axones de petit diamètre à l'intérieur d'une partie spatiale différente de la zone cible.

**FIG. 1**

EP 3 609 574 B1

20

22

CONFIGURE A NERVE MODULATING THERAPY

24

APPLY THE NERVE MODULATING THERAPY TO A
TARGET AREA OF A SUBJECT FOR A TIME

26

TARGET ONE OR MORE SMALL DIAMETER AXONS IN
THE TARGET AREA FOR NEUROMODULATION

# FIG. 2

GANGLION

INTRACELLULAR
ELECTRODE IN B3

INTRACELLULAR
ELECTRODE IN B43

PROXIMAL
RECORDING
ELECTRODE

OPTICAL FIBER

DISTAL RECORDING
ELECTRODE

FIG. 3

LARGE DIAMETER (B3)    SMALL DIAMETER (B43)

I

II

III

LASER OFF        LASER ON        LASER OFF        LASER ON

200 mV
10ms

25μV
10ms

25μV
10ms

FIG. 4

FIG. 5

EP 3 609 574 B1

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2014074176 A1
- US 201428900 A1

- US 2009187223 A1

**Non-patent literature cited in the description**

- Selective control of small versus large diameter axons using infrared laser light (Conference Presentation). **LOTHET EMILIE H et al.** PROGRESS IN BIOMEDICAL OPTICS AND IMAGING. SPIE - INTERNATIONAL SOCIETY FOR OPTICAL ENGINEERING, 08 March 2016, vol. 9690, 96901 N

- Analysis of components of compound action potentials in response to infrared laser light (Conference Presentation). **ZHUO JLTNQI et al.** PROGRESS IN BIOMEDICAL OPTICS AND IMAGING. SPIE - INTERNATIONAL SOCIETY FOR OPTICAL ENGINEERING, 08 February 2017, vol. 10052, 100520N
- **WANG, Y.T. ; ROLLINS, A.M. ; JENKINS, M.W.** Infrared inhibition of embryonic hearts. *J. Biomed. Opt.,* 2016, vol. 21, 60505